# EUROPEAN PATENT APPLICATION

(11) **EP 2 717 057 A1**
(43) Date of publication of application: **09.04.2014**
(21) Application number: 12306212.7
(22) Date of filing: 04.10.2012
(51) Int. Cl.: G01N 33/94, C07H 13/04, C07H 15/26, C07K 16/44

(54) **Morphine-6-glucuronide derivatives, immunogens and immunoassay**

(71) Applicant: Cisbio Bioassays, 30200 Codolet (FR)
(72) Inventor: The designation of the inventor has not yet been filed
(74) Representative: Nevant, Marc

(57) **Abstract**

The invention relates to compounds of formula: 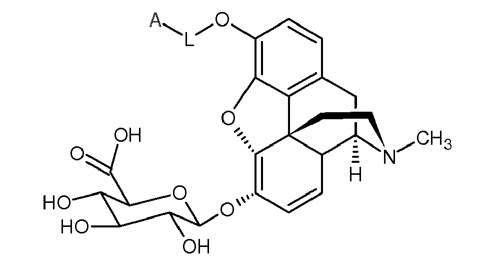 wherein A and L are as defined in the description. The invention also relates to immunogens obtained from said compounds, and to antibodies raised against said immunogens.

The compounds of the invention are used to detect M6G in biological fluids.

## Description

### Background

The present invention relates to Morphine-6-glucuronide (M6G) derivatives and to the use of these compounds for the preparation of immunogens, antibodies and conjugates for use in competitive immunoassays for the detection of M6G. The present invention also relates to a method and kit for detecting or determining M6G.

Morphine-6-glucuronide (M6G) is a metabolite of morphine that can be produced by the brain. It has been demonstrated that this opiate agonist appears to have a greater analgesic potency than morphine. Moreover, Laux et al (J Comp Neurol. 2011 Aug. 15;519(12):2390-416) mapped endogenous morphine-like compounds (morphine, M6G, M3G and codeine) in mouse brain regions not usually involved in pain modulation. Also, international patent application WO 2009/077593 discloses a method of detecting sepsis in a patient, which is based on the detection in patient samples of morphine or naturally occurring metabolites thereof.

Despite the potential clinical interest of M6G, and the amount of work required to assess its clinical significance, no commercial reagents are available as of today to specifically measure this analyte.

Chapman et al (J Pharm Biomed Anal. 1994 Mar;12(3):353-60) evaluated a differential radioimmunoassay technique for the determination of morphine and M6G in samples of human plasma. This technique was based on an antiserum "A" raised against N-succinyl-normorphine-BSA conjugate, specific of morphine, and another antiserum "B" raised against 6-succinyl-morphine-BSA conjugate which cross-reacts with both morphine and M6G. Antiserum A was used with [³H] morphine in a radioimmunoassay, whereas [¹²⁵I]-M6G or [¹²⁵I]-morphine were used with serum B. M6G concentration was calculated by subtracting the results obtained with serum A from the ones obtained with serum B. The authors stressed that in the absence of a specific antiserum for M6G, differential RIA remains the only immunoassay technique available in 1994 for the determination of M6G.

The same authors later described a specific radioimmunoassay for the determination of M6G in human plasma (Ann Clin Biochem. 1995 May;32:297-302). An antiserum was raised by immunization of rabbits with *N*-aminobutyl-normorphine-6-glucuronide conjugated with thyroglobulin. According to the authors, their radioimmunoassay could theoretically detect specifically M6G down to 0.05 ng/mL without interference from M3G or morphine, but unfortunately this antiserum is not commercially available. The synthesis of the above-mentioned conjugate requires 12 steps and was poorly described by Brown et al (Tetrahedron Letters, Volume 36, Number 47, 20 November 1995, pp. 8661-8664(4)).

Currently, the only way to specifically measure M6G in a biological sample is by mass spectrometry. Anti-M6G antibodies that do not significantly cross-react with morphine and M3G are not commercially available, and the same holds true for immunoassays that specifically measure M6G from a biological sample. There is therefore a need for tools and methods to specifically detect and determine M6G in biological samples.

### Description of the drawings

Figure 1 shows the optical density (representing the quantity of M6G-HRP bound to the antibody) as a function of the concentration of competing antigen.
Figure 2 shows the percentage of patients with detectable levels of M6G in various groups of patients.

### Description of the invention

It is an object of the invention to overcome some or all of the disadvantages of the prior art.

New derivatives of M6G have been found and are the object of the invention : these compounds are particularly useful to manufacture immunogens which are another object of the invention and that can be used to obtain antibodies which are specific of M6G and which do not significantly cross-react with morphine, M3G and codeine. These antibodies constitute yet another object of the invention. By "not significantly" is meant a cross-reactivity of less than about 25%, preferably less than about 10%, more preferably less than about 7.5%, still more preferably less than about 5% when compared to 100% for M6G. "Not significantly" also means that the affinity constant of M6G for the antibody is at least around 20 times better, i.e. 20 times lower, preferably at least 30 times better than that of potentially cross-reacting antigens.

In order to achieve such specificity, the compounds described in the present invention are generated by introduction of a substituent bearing a functional group at the carbon 3 of the morphinan ring system. These compounds can also be used to manufacture labeled M6G, i.e. conjugates of M6G with a detectable tracer that can be used for example in immunoassays. These conjugates are another object of the invention.

Finally, other objects of the invention are a method and a kit for detecting, or determining the quantity of M6G. By "detecting" is meant qualitatively analyzing for the presence or absence of a substance. By "determining" is meant quantitatively analyzing for the amount of a substance.

In a first aspect, the inventions is directed to M6G derivatives bearing a functional group connected via a divalent linker to the carbon 3 of the morphinan ring system of M6G. These derivatives are compounds of formula (I): 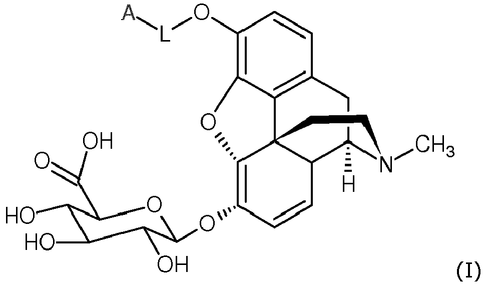 wherein L is a divalent linker and A is a functional group for coupling compound (I) to either an antigenicity-conferring carrier material, a detectable labeling agent, or a solid support.

Preferably, the divalent linker L is chosen from the group consisting of : 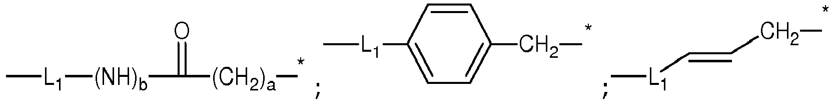 wherein a and b are independently 0 or 1;
the asterisk denotes the point of attachment to the oxygen atom of the morphinan ring;

L₁ is linked to group A and is chosen from: a covalent bond; a linear or branched, saturated or unsaturated C₁-C₂₀ alkylene moiety optionally containing one or more heteroatoms, such as oxygen, nitrogen, sulfur, phosphorus or one or more carbamoyl or carboxamido groups; a saturated or unsaturated C₅-C₈ cycloalkylene moiety; a saturated or unsaturated C₆-C₁₄ arylene moiety; said alkylene, cycloalkylene or arylene moieties being optionally substituted with alkyl, aryl or sulfonate groups. Suitable C₅-C₈ cycloalkylene moieties include cyclohexylene. Suitable C₆-C₁₄ arylene moieties include phenylene and xylylene.

### In particular, L1 is chosen from:

1)

   -(CH₂)ₙ-
2) 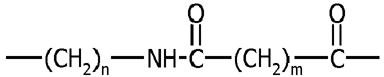
3) 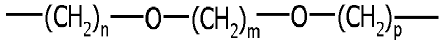
4) 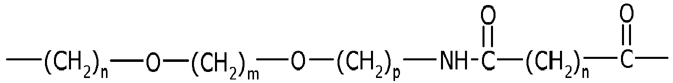
5) 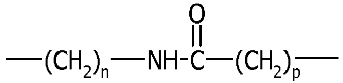
6) 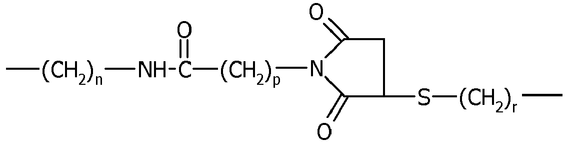
7) 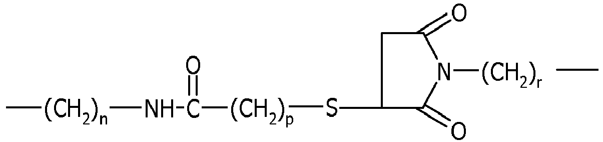
8) 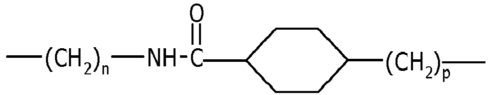
9) 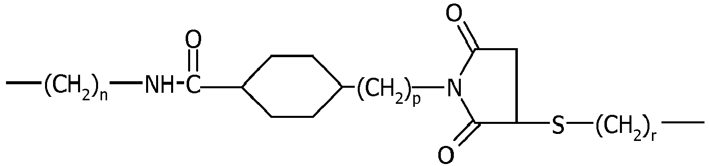
10)

   -(CH₂)ₙ-NH-
11) 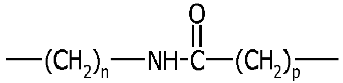
12) 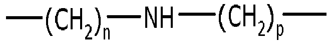
13) 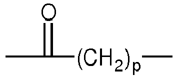 in which n, m, p, r are each independently an integer of 1 to 16, preferably of 1 to 5.

Advantageoulsy, L is a group of formula: 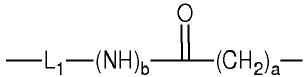 where a=b=1 and where L1 is a C₁-C₁₀ alkylene moiety, preferably a C₂-C₆ alkylene moiety.

A is preferably chosen from: an acrylamide, an activated amine (for example a cadaverine or an ethylenediamine), an activated ester, an aldehyde, an alkyl halide, an anhydride, an aniline, an azide, an aziridine, a haloacetamide, a halotriazine, such as monochlorotriazine, dichlorotriazine, a hydrazine (including hydrazides), an imido ester, a maleimide, a sulfonyl halide, a thiol, a ketone, an amine, a hydroxysuccinimidyl ester, a hydroxysulfosuccinimidyl ester, an ethynyl fragment, an azidonitrophenyl, an azidophenyl, a 3-(2-pyridyldithio)-propionamide, glyoxal and the groups of formula: 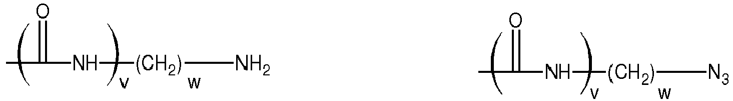 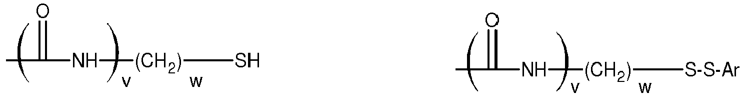 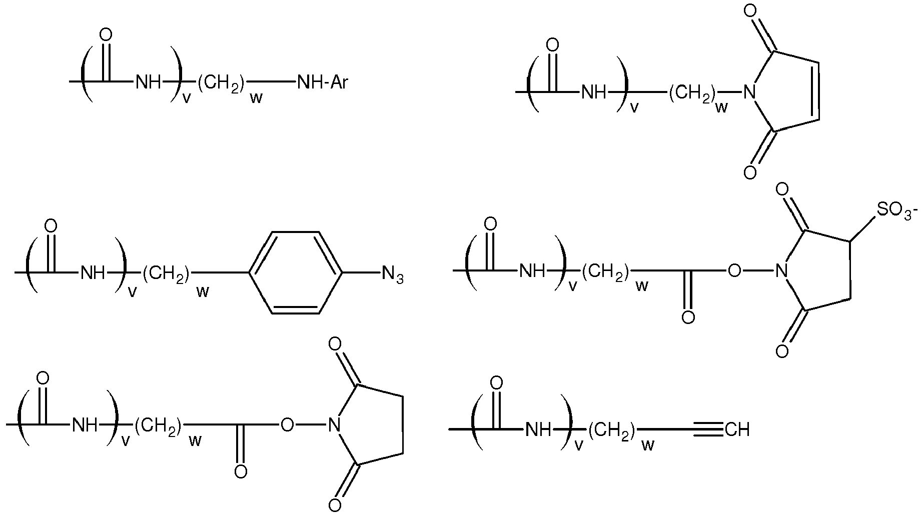 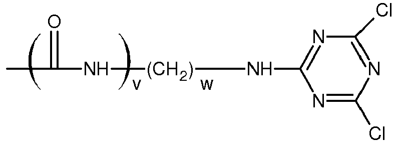 where w varies from 0 to 8 and v is equal to 0 or 1, and Ar is a 5- or 6-membered, saturated or unsaturated heterocycle comprising 1 to 3 heteroatoms, said heterocycle being optionally substituted with a halogen atom.

Preferably, A is a carboxylic acid succinimidyl ester, a haloacetamide, a hydrazine, a maleimide group, an amine, and more preferably A is -NH₂.

A preferred group of compounds include the compounds of formula (I) where L is a group of formula: 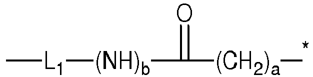 where a=b=1 and where L₁ is a C₁-C₁₀ alkylene moiety, preferably a C₂-C₆ alkylene moiety and A is as defined above, and is preferably -NH₂.

A particularly preferred compound is the compound (II):

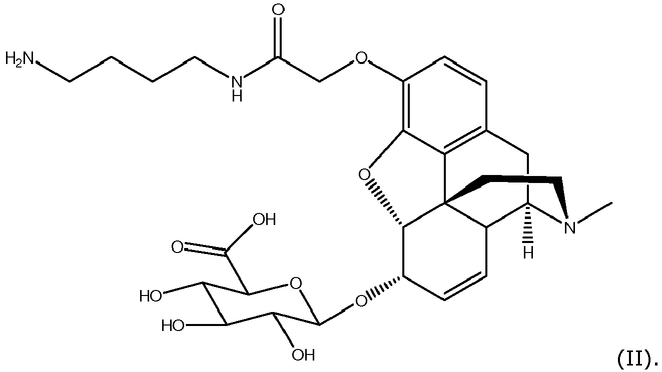

Examples of suitable Ar groups include the following groups: pyrrolyl, pyrazolyl, imidazolyl, oxazolyl, isoxazolyl, triazolyl, oxadiazolyl, furanyl, thienyl, thiazolyl, isothiazolyl, thiadiazolyl, pyridyl, pyridazinyl, pyrimidinyle pyrazinyl, triazinyl, pyrrolidinyl, tetrahydrofuryl, tetrahydrothienyl, pyrrolidinyl, imidazolidinyl, thiazolidinyl, isoxazolidinyl, piperidinyl, piperazinyl, morpholinyl, thiomorpholinyl.

Unless otherwise indicated, the term "a" or "an" (for example as in the expression "an antibody") is intended to mean "at least one".

The functional groups A are used for coupling the M6G derivatives of the present invention to carrier materials for the preparation of the corresponding immunogens. The resulting immunogens can be administered to hosts to elicit production of avid specific antisera, preferably polyclonal antisera, which are then used to develop sensitive immunoassays for the detection of M6G. Surprisingly, the antibodies obtained with such immunogen do not significantly cross-react with morphine, M3G and codeine.

The invention, therefore, also provides an immunogen comprising an M6G derivative according to the present invention, coupled to an antigenicity-conferring carrier material. Preferably, the carrier material is a protein, a protein fragment, a synthetic polypeptide or a semi-synthetic polypeptide, even more preferably chosen from the group consisting of: bovine serum albumin (BSA) or cationic BSA (cBSA), KLH (Keyhole Limpet Haemocyanin), thyroglobulin, ovalbumin. The carrier molecule may also be non-proteic and chosen from the group consisting of: liposomes, polymers of L-lysine or of L-glutamic acid, ficoll, dextran, or polyethylene glycol. Carrier molecules are commercially available and generally contain functional groups which will react with the functional group A of the M6G derivatives of the invention, or alternatively such groups may be introduced by conventional techniques. The conjugation of amine-containing derivatives of the invention with a carrier protein in particular, can be achieved by a glutaraldehyde condensation reaction.

In a further aspect, the present invention provides antibodies raised against the immunogens of the present invention. Preferably, the antibodies are polyclonal. Alternatively, the antibodies are monoclonal. The term "antibody" also includes single chain variable fragments (scFv), single domain antibodies (sdab, also known as nanobodies) as well as antibody fragments comprising at least one variable domain (such as F(ab')₂, Fab).

In a still further aspect, the present invention provides a conjugate comprising the M6G derivative of the present invention covalently bonded to a detectable labeling agent. Preferably, the labeling agent is selected from an enzyme, a luminescent substance, or a radioactive substance. More preferably, the labeling agent is an enzyme, preferably a peroxidase, most preferably horseradish peroxidase (HRP). Alternatively, or additionally, the luminescent substance may be a bioluminescent, chemiluminescent or fluorescent material. Luminescent or enzymatic labeling agents are commercially available and are provided in a form suitable for conjugation with the M6G derivatives of the invention, i.e. they contain a functional group which will react with the functional group A of the M6G derivatives of the invention. The following labeling agents are particularly preferred since they are commonly used in biological analysis:
*Enzymatic labels*: horseradish peroxidase (HRP), luciferase. Those enzymes are commercially available in a form suitable for conjugation with the compounds of the invention.
*Fluorescent labels*: rhodamines, cyanines, squaraines, fluorophores known under the name BODIPY, fluoresceins, compounds known under the name AlexaFluor, rare-earth chelates, rare-earth cryptates, quantum dots, phycobiliproteins, such as B-phycoerythrin, R-phycoerythrin, C-phycocyanin, allophycocyanins. Again, those compounds are commercially available in a form that is suitable for conjugation with the M6G derivatives of the invention.

Alternatively, the labeling agent is a member of a pair of binding partners. Such binding partners may be one of the following pairs: avidin or streptavidin/biotin, DNP/anti-DNP antibodies. Consequently, the invention also comprises a conjugate of the M6G derivative of the invention, covalently bonded with biotin or DNP. Addition of the second member of the binding partner pair, itself labeled with a fluorescent or enzymatic tracer, will ensure the labeling of M6G with a detectable compound.

In another aspect, the invention provides a conjugate comprising the M6G derivatives of the invention covalently linked to a solid support. In this case, the solid support can be a magnetic or non-magnetic microbead, the well of a microplate, a tube, a fluorescent microbead, or any other solid phase system necessary for a detection system. Coating or conjugation of the M6G derivatives of the invention with solid supports can be done with standard techniques, due to the presence of the functional group A.

The invention further provides a process of preparing anti-M6G antibodies, the process comprising the steps of immunizing an animal, preferably a vertebrate animal, most preferably a mammalian animal, by repeated administration of an immunogen according to the present invention, and collecting the resulting serum from the immunized animal. Antibodies prepared in accordance with this process are polyclonal.

Alternatively, the process further comprises removing the animal's spleen and fusion of a mixture of lymphocytes and plasmocytes from this spleen with myelomatous cells in the presence of an inducer of cell fusion, such as polyethylene glycol, in order to obtain hybdridoma producing monoclonal antibodies.

In a further aspect, the present invention provides a method for detecting or determining M6G in a sample, the method comprising contacting the sample with the conjugate of the present invention, and with antibodies of the present invention; detecting or determining bound conjugate; and deducing from a calibration curve the presence of, or the amount of, M6G in the sample.

In a still further aspect, the invention provides a kit for detecting or determining M6G, the kit including the conjugate of the present invention and the antibodies of the present invention. The kit may optionally include instructions for the use of said conjugates and said antibodies for detecting or determining M6G in a sample.

Preferably, the sample is a solution, such as a biological fluid. More preferably, the sample is plasma, serum or urine. Most preferably, the sample is a solution from a human patient.

In a further aspect, the present invention provides the use of the conjugates according to the present invention with the antibodies according to the present invention, to detect or determine M6G in samples such as biological fluids.

### Preparation of M6G derivatives bearing a functional group for conjugation:

M6G is commercially available and a convergent and cost-efficient synthesis has been used in order to introduce an arm on carbon 3 of M6G. Condensation of bromoacetyl bromide on Fmoc monoprotected butadiamine led to the expected linker, which could be reacted with M6G in the presence of potassium carbonate in an ethanol solvent. These conditions allowed the one step synthesis of the desired M6G-3-NH₂, since they also ensured the removal of the Fmoc protective group.

### Preparation of Immunogens and Conjugates

Although the M6G derivatives of the present invention provide defined structural epitopes, they are not in themselves immunogenic and therefore need to be conjugated to a carrier material which will elicit an immunogenic response when administered to a host animal. Suitable carrier materials commonly contain poly(amino acid) segments and include polypeptides, proteins such as albumins, serum proteins e.g. globulins and ocular lens proteins and lipoproteins. Illustrative protein carrier materials include bovine serum albumin (BSA), egg ovalbumin, bovine gamma globulin, thyroxin binding globulin, keyhole limpet haemocyanin (KLH) etc. Alternatively, synthetic poly(amino acids) having a sufficient number of available amine groups such as lysine may be employed, as may other synthetic or natural polymeric materials bearing reactive functional groups. In particular, carbohydrates, yeasts or polysaccharides may be conjugated to the M6G derivatives of the present invention to produce immunogens of the present invention.

Each M6G derivative of the present invention can also be coupled to a labeling agent such as an enzyme (for example, horseradish peroxidase), a substance having fluorescent properties or a radioactive label for the preparation of conjugates (or detection reagents) for use in the immunoassays. The fluorescent substance may be, for example, a monovalent residue of fluorescein or a derivative thereof.

In preparing immunogens or conjugates with M6G derivatives of the present invention where a thiol group is present, maleimide, halo or vinylsulphone groups must first be introduced to the carrier material or labeling agent using heterobifunctional linkers such as: *N*-(γ-maleimidobutyryloxy)succinimide ester (GMBS); succinimidyl 4-(N-maleimidomethyl)cyclohexane-1-carboxylate (SMCC); (m-maleimidobenzoyl)-N-hydroxysuccinimide (MBS); succinimidyl 4-(p-maleimidophenyl)butyrate (SMPB); N-succinimidyl(4-iodoacetyl)aminobenzoate (SIAB); bromoacetylglycine N-hydroxysuccinimide; N-succinimidyl 3-(2-pyridyldithio)propionate (SPDP); or vinylsulphone (Pierce Chemical Company, USA). The thus-modified carrier material or labeling agent can then be conjugated via the thiol groups on the M6G derivative. For M6G derivatives without a thiol group, such as the compound of formula (II) of the present invention, conjugation is performed without prior modification of the carrier material or labeling agent using standard methods of conjugation such as glutaraldehyde condensation, mixed anhydride, EDC or succinimidyl activation of the M6G derivative. Conjugation methods are well known to those skilled in the art (Bioconjugate Techniques, G. T. Hermanson, Academic Press, Second Edition 2008).

### Preparation of antibodies

In order to generate polyclonal antisera, each immunogen of the present invention is mixed with Freund's Adjuvant and the mixture is injected into a host animal, such as a rabbit, sheep, mouse, guinea pig, Ilama or horse. Further injections (boosts) are made and serum is sampled for evaluation of antibody titer. When the optimal titer has been reached, the host animal is then bled to yield a suitable volume of specific antiserum. The degree of antibody purification required depends on the intended application. For many purposes, there is no requirement at all for purification, however, in other cases, such as where the antibody is to be immobilized on a solid support, purification steps can be taken to remove undesired material and eliminate non-specific binding.

Alternatively and in order to generate monoclonal antibodies, the animal spleen is removed. A mixture of lymphocytes and plasmocytes from this spleen is fused *in vitro* with myelomatous cells in the presence of an inducer of cell fusion, such as polyethylene glycol. A mutant myelomatous cell line, lacking hypoxanthine guanosin phosphoribosyl transferase (HGPRT), is used to allow easy selection of the hybrid cells. These cells are cultured in a medium containing hypoxanthine, aminopterin (methotrexate) and thymine (HAT medium) in order to eliminate the non-fused myelomatous cells and select the hybridomas of interest. The non-fused spleen cells die because they are not capable of proliferating *in vitro.* The hybrid cells, on the other hand, survive. The hybridomas thus obtained are cultured in the wells of a cell culture plate. The supernatants of these wells are tested for the presence of anti-M6G antibodies specific in a RIA- or ELISA-based screening test. The hybridomas are then cloned and may be injected into mammals in order to induce myelomas secreting the anti-M6G antibody in a large quantity in ascitic fluid.

The antibodies of the invention also include anti-M6G antibody fragments such as F(ab')₂ fragments, which can be generated by pepsin digestion of an intact antibody molecule, and Fab fragments, which can be generated by reducing the disulfide bridges of the F(ab')₂ fragments.

Alternatively, Fab, scFv (single chain fragment antibodies) or sdab (single domain antibodies) expression libraries can be constructed to allow rapid identification of fragments having the desired specificity to M6G. For example, phage display of antibodies may be used. In such a method, scFv, Fab or sdab fragments are expressed on the surface of a suitable bacteriophage, e.g., M13. The coding regions of the VL and VH chains are obtained from circulating lymphocytes, spleen cells or lymph nodes from mammals immunized with the immunogen of the invention. These coding regions are then fused to a terminus of a phage sequence. Once the phage is inserted into a suitable carrier, e.g., bacteria, the phage displays the antibody fragment, and those binding to M6G can be isolated.

The antibodies of the present invention are useful as reagents in biochemical assays for the detection or determination of M6G in biological fluids.

### Examples

### Reagents and analytical methods:

Morphine, normorphine, codeine, morphine-3-glucuronide (M3G), keyhole limpet haemocyanin (KLH), glutaraldehyde, triethylamine (TEA), bovine serum albumin (BSA) protease free, avidin, dimethylformamide anhydrous (DMF), *N*-Fmoc-1,4-butadiamine hydrobromide, bromoacetyl bromide, triethylamine, potassium carbonate, diisopropylethylamine, dimethylsulfoxide, dichloromethane were purchased from Sigma-Aldrich (St Louis, USA). Morphine-6-glucuronide (M6G) was from Carlo Erba Réactifs-SDS (Val de Reuil, France). Sodium borohydride (NaBH₄) was from Acros (Geel, Belgium). Biotin-NHS was from Calbiochem (Darmstadt, Germany). H₂SO₄ was obtained from Merck (Darmstadt, Germany). StabilZyme® HRP Conjugate Stabilizer was from Surmodics (Eden Prairie, USA). Chromogenic substrate TMB (3,3',5,5'-tetramethylbenzidine) was from KPL (Maryland, USA). 96-well microplates were from NUNC (Roskilde, Denmark). New born calf serum (NBCS) was from PAA laboratories (Linz, Austria). Activated horseradish-peroxidase (HRP) was from Roche Diagnostics (Meylan, France). HiTra™ Protein A HP Columns and Sephadex HR10/30 were from GE Healthcare lifescience (Chalfont St Giles, UK). Dialysis tubing and AMICON ultra pore were from Millipore (Molsheim, France). Biotinamidocaproate-N-hydroxysuccinimide ester was supplied by Pierce. Solvents were purchased from Carlo Erba and were used without further purification (MeCN = acetonitrile, AcOEt = ethyl acetate). Other chemicals compounds for buffer preparation were from Merck and Sigma-Aldrich.

For Thin-layer chromatography (TLC) silica gel plates 60 F254 were used and compounds were visualized by UV light, iodine or phosphomolybdic acid. ¹H and ¹³C spectra were recorded in DMSO-d6, on Bruker Avance III 400 MHz spectrometer. Chemical shifts are reported as δ values in parts per million relative to TMS as an internal standard. Splitting pattern abbreviations are as follows: s = singlet, d = doublet, dd = double doublet, ddd = a doublet of doublet of doublets, t = triplet, td = triplet of doublets, m = multiplet. Coupling constants are in Hertz. UV spectra were determined on Beckman Coulter DU 800 spectrophotometer. Reactions were monitored by LC-MS (low resolution mass spectrometry) on Waters alliance 2695 coupled with Micromass ZQ mass spectrometer (ESI+). HRMS was performed with an Applied Biosystems SCIEX QStar Elite using for ionization an (ESI+) mode and a TOF for analyser. The analysis was done in triplicate. Melting points were measured using Buchi Melting point B-540. Purifications were carried out by column chromatography on silica gel (Merck, 60-200 µm, porosity 60 Å). The analytical HPLC (High-performance liquid chromatography) was performed on a Thermoscientific Spectra System P1000XR equipped with diode array detector UV1000. The preparative HPLC was performed on a Shimadzu LC-8A equipped with UV/vis detector SPD. Several chromatographic systems were employed for analytical and preparative HPLC:
System A: HPLC (Waters XBridge RP-C₁₈ column, 3.5 µm, 4.6 × 100 mm) with 0.2% aq. Trifluoroacetic acid (pH 1.- MeCN (v/v) as eluents [isocratic 5% MeCN (2 min), linear gradient from 5 to 100% MeCN (18 min), at a flow rate of 1 mL.min⁻¹] and UV detection at 280 nm.
System B: : HPLC (Waters XBridge RP-C₁₈ column, 5 µm, 19 × 100 mm) with 0.1% aq. formic acid (pH 2.8 - MeCN (v/v) as eluents [isocratic 2% MeCN (3.5 min), linear gradient from 2 to 100% MeCN (16.5 min), at a flow rate of 20 mL.min⁻¹] and UV detection at 280 nm.
System C: HPLC (Waters XBridge RP-C₁₈ column, 5 µm, 19 × 100 mm) with 0.1% aq. formic acid (pH 2.8 - MeCN (v/v) as eluents [isocratic 2% MeCN (2 min), linear gradient from 2 to 50% MeCN (17 min), linear gradient from 50 to 100% MeCN (1 min) at a flow rate of 20 mL.min⁻¹] and UV detection at 280 nm.

### Example 1: Preparation of M6G-3 derivatives

### (9H-fluoren-9-yl)methyl (4-(2-bromoacetamido)butyl)carbamate

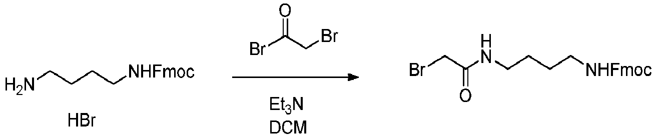

To a suspension of *N*-Fmoc-1,4-butadiamine hydrobromide (196 mg, 0.5 mmol) in dichloromethane (10 mL) was added at room temperature triethylamine (0.2 mL, 1.5 mmol). To the resulting solution cooled to -10°C, was added dropwise a solution of bromoacetyl bromide (43 µL, 0.5 mmol) in dichloromethane (1 mL) over a 10 min period under an inert atmosphere. The reaction was monitored by TLC (dichloromethane-methanol, 90/10) and after this period the reaction was completed. To this mixture was added a saturated solution of sodium carbonate (10 mL). The aqueous phase was extracted with dichloromethane (10 mL), the organic phases were combined, dried over magnesium sulfate, filtered and the solvent was removed *in vacuo.* The resulting brown residue was purified by flash chromatography on silica (dichloromethane-methanol 99/1 to 97/3 in 1% increment) to give the desired compound as a white solid (125 mg, 58%). Mp 154-155°C. ¹H NMR (400 MHz, DMSO): δ = 8.25 (t, *J* = 4.6 Hz, 1H), 7.89 (d, *J* = 7.5 Hz, 2H), 7.69 (d, *J* = 7.5 Hz, 2H), 7.42 (t, *J* = 7.3 Hz, 2H), 7.32 (t, *J* = 7.3 Hz, 2H), 7.29 (t, *J* = 5.6 Hz, 1H), 4.30 (d, *J* = 6.9 Hz, 2H), 4.21 (t, *J* = 6.8 Hz, 1H), 3.83 (s, 2H), 3.05-3.10 (m, 2H), 2.95-3.05 (m, 2H), 1.35-1.45 (m, 4H). NMR ¹³C (100 MHz, DMSO): δ 166.34; 156.58, 144.39, 141.20, 128.07, 127.52, 125.61, 120.58, 65.64, 47.24, 40.37, 39.22, 30.03, 27.25, 26.60. HRMS (ESI): calcd. for C₂₁H₂₄BrN₂O₃ (M+H)⁺, 431.0970; found 431.0968. TLC: R*f:* 0.57 silica, DCM/MeOH, 90/10.

### M6G-3-NH₂

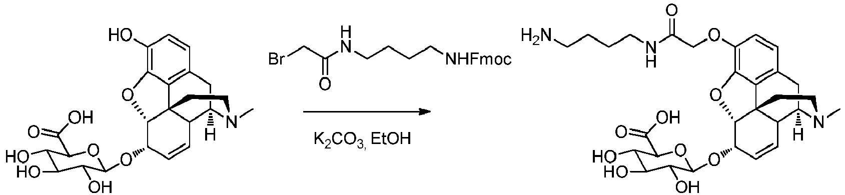

To a solution of M6G (0.5 mg, 1 µmol) in dry ethanol (1 mL), were added (9H-fluoren-9-yl)methyl (4-(2-bromoacetamido)butyl)carbamate (0.9 mg, 2 µmol) and carbonate potassium (0.4 mg, 3 µmol). The resulting suspension was heated at reflux for 1.5 h under an inert atmosphere. The reaction was monitored by analytical HPLC using system A. After this period the reaction was completed. The mixture was allowed to reach room temperature and the solvent was removed *in vacuo.* The crude product was diluted in a mixture of methanol (200 µL) and water containing 0.2% trifluoroacetic acid (8 mL) and the resulting solution was purified by preparative HPLC using system B to give the desired M6G-NH₂ as a white solid (0.26 mg, 52%). HRMS (ESI): calcd. for C₂₉H₄₀N₃O₁₀ (M+H)⁺, 590.2714; found 590.2703. HPLC *t_{R}* (*System A*) = 6.21 min.

### M6G-3-Biotine

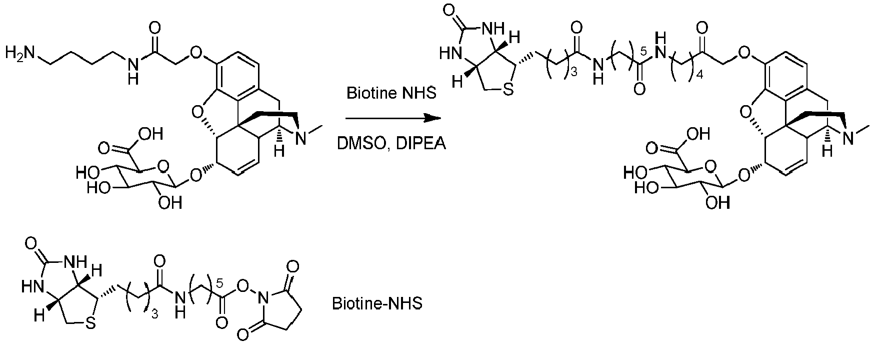

To a solution of M6G-NH₂ (1.2 mg, 2.2 µmol) in dry dimethylsulfoxide (500 µL) protected from UV light were added biotinamidocaproate-*N*-hydroxysuccinimide ester (1 mg, 2.6 µmol) and diisopropylethylamine (0.5 µL, 2.8 µmol) under an inert atmosphere. The mixture was stirred at room temperature for 1 h. The reaction was monitored by analytical HPLC using system A. After this period the reaction was completed. The resulting solution was purified by preparative HPLC using system C to give the desired M6G-3-biotine (1.56 µmol, 70%). HRMS (ESI): calcd. for C₄₅H₆₅N₆O₁₃S (M+H)⁺, 929.4330; found 929.4323. HPLC *t_{R}* (*System A*) = 7.88 min.

### Example 2: Preparation of M6G immunogen

The M6G-3-NH₂ hapten was coupled to KLH by glutaraldehyde condensation reaction using a molar ratio of 50:1 for hapten: protein. 832 nmol of hapten diluted in phosphate buffer saline (PBS) were added to 16.5 nmol of KLH leading to a mixture of 1.2 mL. Then 144 µL of glutaradehyde 10% were added and the mixture was stirred 2 h at room temperature (RT). The reaction was stopped with NaBH₄. The resulting conjugate was then dialyzed against PBS (2 x 2L) over 24 h, aliquoted and stored at -20°C.

### Example 3: Preparation of antisera

### Immunization protocol

The immunogen described above was diluted in PBS pH 7 (1 mg.mL⁻¹), mixed with complete Freund's adjuvant and injected subcutaneously in two males New Zealand rabbits. A first boost was given at day 30 after the initial inoculation and subsequent boosts were given every 30 days. Blood was taken after the second boost from the marginal ear vein and after a one week interval following each immunization. The anti-serum obtained was stored at -20°C. The titer of the rabbit antiserum was monitored by ELISA. The antiserum of rabbit 242 had the highest titer and was then were purified using HiTrap™ Protein A HP Columns according to manufacturer instructions.

### Antibody, standard and controls preparation

The rabbit anti-M6G polyclonal antibody was biotinylated for the ELISA assay. After dialysis in buffer carbonate 100 mM pH 9, biotin-NHS dissolved in DMF was added to the antibody. After stirring for 30 min, the free biotin was removed by dialysis in PBS 11 mM pH 7.2. Aliquots of labeled antibody were frozen and stored at -20°C. Then, the biotinylated antibody was diluted at ELISA working concentration determined at 0.25µg.mL⁻¹ in PBS 11 mM pH 7.2 + 1% BSA, aliquoted and stored at -20°C.

Calibration curves were prepared in NBCS by spiking M6G over the range from 0 to 20 ng.mL⁻¹.

2 controls with 2 different M6G concentrations were prepared using normal serum for control 0 and pool of pathological serum for control C, aliquoted and stored at -20°C.

### Example 4: Preparation of M6G-HRP

The M6G-3-NH₂ hapten was coupled to HRP to synthesize a M6G ELISA tracer. A molar ratio of 5:1 was used (hapten:protein). Conjugation was done according to the manufacturer instructions. Briefly, 27 µg of M6G-3-NH₂ was added to 1 mg of activated HRP in sodium carbonate buffer 1 M pH 9.4. The mixture was stirred overnight at 4°C. To stop the reaction, 40 µL of TEA 2M pH8 and 50 µL of NaBH₄ were added. The mixture was stirred for 30 min at 4°C. 25µL of TEA were added again and the final mixture was incubated for 2 h at 4°C. Finally, 10 µL of glycine 1 M, pH 7 were added to stabilize the conjugate. Purification of the conjugate was done using gel filtration Superdex HR10/30. Finally the conjugate was diluted 30 fold in Stabilzyme®, aliquoted and stored at 4°C. For ELISA assays, the conjugate was diluted at working concentration (100 ng.mL⁻¹) in Stabilzyme®, aliquoted and stored at 4°C.

### Example 5: M6G immunoassay

### Microplates preparation

96-well microplates were coated with 200 µL/well of a 10 µg.mL⁻¹ BSA-biotin solution in PBS and incubated for 24 h at room temperature. Then after aspiration 200 µL/well of 10 µg.mL⁻¹ of avidin solution in PBS were distributed and incubated for 24 h at room temperature. After washing with NaCl, Tween® 0.005%, 200 µL/well of saturation solution was added (PBS + 2% Sorbitol + 0.5% Glycine + 0.1% BSA + 0.065% NaN₃) and incubated for 24 h at 4°C. After an aspiration, the microplates were dried at 20°C in a ventilated room for 12 h, packed with a dessicant and stored at 4°C.

### Assay procedure

A competitive assay format was used for the M6G-ELISA. 100 µL of biotinylated antibody were added and incubated for 2 h at room temperature under 400 rpm stirring. After incubation, the wells were emptied and 3 wash cycles with H₂O + 0.05% Tween® were applied. 50 µL of calibrators, controls and samples were added in duplicate. If necessary, samples were prediluted in dilution buffer (PBS + 0.1% BSA), then 50 µL of M6G-HRP conjugate was distributed in all the wells and incubated for 2h at room temperature, under 400rpm stirring. After a final washing step, 100 µL of HRP substrate (TMB) were added, and incubated for 10 min at room temperature, under 400rpm stirring. The chromogenic reaction was stopped with 100 µL H₂SO₄ 1 N, and the absorbance of each sample was determined at 450 nm and 620 nm (reference wavelength). The results were analyzed using GraphPad Prism Software, model one site competition.

### Exemple 6: Cross-reactivity of the M6G-ELISA

The cross-reactivity of the assay described in example 5 with several compounds that are structurally close to M6G was evaluated. For this purpose, the assay was performed with 50 µL of samples constituted of competing compounds diluted in NBCS at the concentrations of: 1 nM, 10 nM, 100 nM or 1000 nM. The following compounds were tested:
*Morphinic Alcaloïds*: Morphine-6-glucuronide (CAS [20290-10-2]), Morphine (CAS [57-27-2]), Morphine-3-glucuronide (CAS [20290-09-9]), Codeine (CAS [76-57-3]), Morphine-3-6-diglucuronide (CAS [64947-41-7]), Norcodeine (CAS [467-15-2]), Normorphine (CAS [466-97-7]).
*Monoamines and catecholamines* : Dopamine (CAS [51-61-6]), Adrenaline (CAS [51-43-4]), Noradrenaline (CAS [51-41-2]), L-DOPA (CAS [59-92-7]), DOPAC (CAS [102-32-9]), THP (tetrahydropapaveroline CAS [47-99-3]).
*Peptidic agonists*/*antagonists of the opioid receptors*: DAGO ([CAS [78123-71-4]), CTOP (CAS [103429-31-8]), CTAP (CAS [103429-32-9]).
*Semi-synthetic antagonists of the opioid receptors*: Naloxonazine (CAS [82824-01-9]), Naltrexone (CAS [16676-29-2]), Naloxone (CAS [357-08-4]), Naloxone methiodide (CAS [93302-47-7]), Naltrindole (CAS [111469-81-9]), 3-MethoxyNaltrexone (CAS [16617-07-5]).
*Synthetic agonist of* µ*-opioid receptors (used in clinical applications)* : Fentanyl (CAS [437-38-7]).

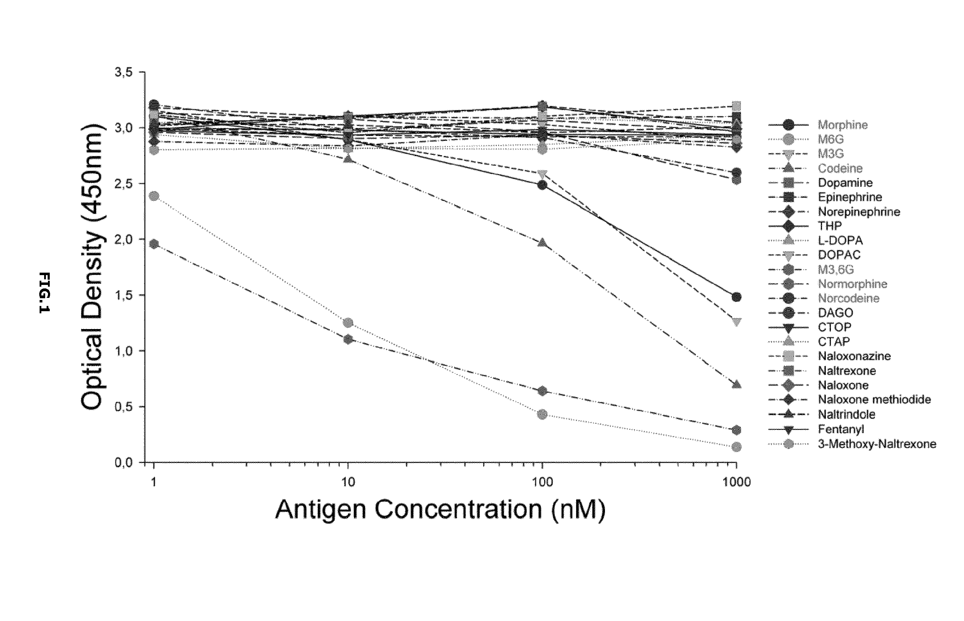

The results are presented in Figure 1. Approximate affinity constants have been calculated from the figure:
- Strong cross-reactivity is only observed with M6G (K=5.9 nM) and M3,6G (K=3.4 nM), which was expected since M3,6G displays the same paratope than M6G. This is however not a problem considering that M3,6G constitute less than 1 % of the products generated through catabolism of exogenous morphine.
- Non-significant cross-reactivity has been observed with codeine (K=0.22µM, i.e. 35X as high as M6G), M3G (K=0.67µM i.e. more than 100X as high as M6G), morphine (K=0.97µM i.e. more than 160X as high as M6G), normorphine (K >>1µM), norcodeine (K >> 1µM).
- The other compounds do not cross-react with the antibody

This example shows that the antibody and the kit of the invention are extremely specific of M6G and also very sensitive with a detection limit lower than 1 nM (for M6G, 1 nM=0.462 ng.mL⁻¹).

### Example 7: Use of the M6G immunoassay with patient samples

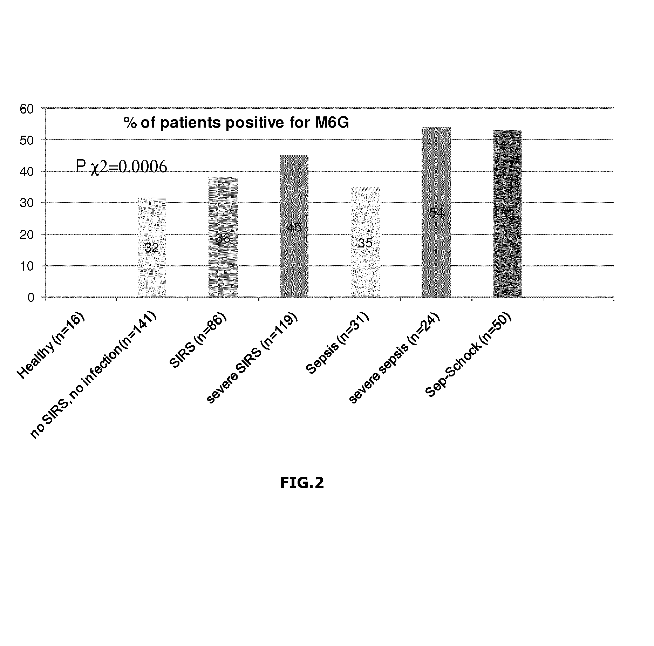

The assay M6G-ELISA of example 5 has been used with serum from patients. The results are presented in figure 2 which shows the percentage of patients with detectable levels of morphine-6-glucuronide (M6G) in different groups of patients: healthy volunteers; patients with neither systemic inflammatory response syndrome (SIRS) nor infection; patients with SIRS; patients with severe SIRS (as defined in Intensive Care Med 2008;34:1654-61); patients with sepsis; patients with severe sepsis; and patients with septic shock (the last three conditions being as defined in Chest 1992;101:1481-1483). There was a larger proportion of patients with SIRS and Sepsis presenting with detectable levels of M6G than in healthy controls (no subject with detectable levels). There was also an association with the severity with a larger proportion of detectable M6G in patients with severe sepsis and sepsis shock compared to patients with sepsis only.

## Claims

1. A compound of formula (I): 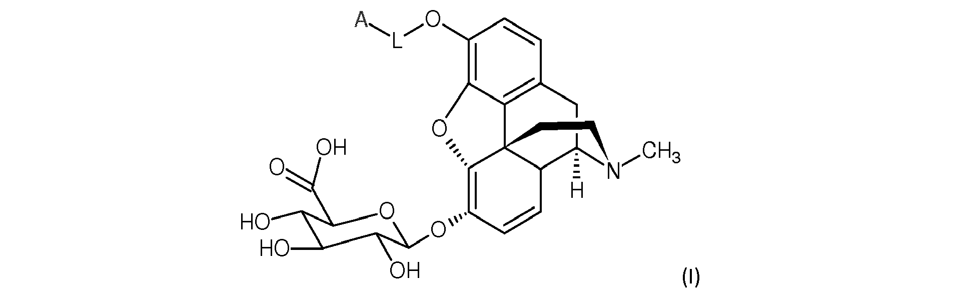 wherein L is a divalent linker and A is a functional group for coupling said compound to either an antigenicity-conferring carrier material, a detectable labeling agent, or a solid support.

2. The compound according to claim 1, wherein L is chosen from the group consisting of : 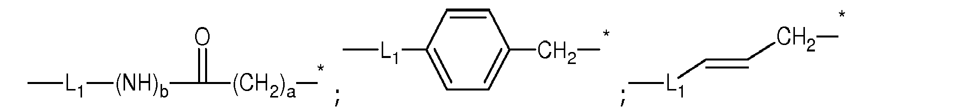 wherein a and b are independently 0 or 1;
the asterisk denotes the point of attachment to the oxygen atom of the morphinan ring;
L₁ is linked to group A and is chosen from: a covalent bond; a linear or branched, saturated or unsaturated C₁-C₂₀ alkylene moiety optionally containing one or more heteroatoms or one or more carbamoyl or carboxamido groups; a saturated or unsaturated C₅-C₈ cycloalkylene moiety; a saturated or unsaturated C₆-C₁₄ arylene moiety; said alkylene, cycloalkylene or arylene moieties being optionally substituted with alkyl, aryl or sulfonate groups.

3. The compound according to claim 1 or 2, wherein A is chosen from: an acrylamide, an activated amine, an activated ester, an aldehyde, an alkyl halide, an anhydride, an aniline, an azide, an aziridine, a haloacetamide, a halotriazine, a hydrazine, an imido ester, a maleimide, a sulfonyl halide, a thiol, a ketone, an amine, a hydroxysuccinimidyl ester, a hydroxysulfosuccinimidyl ester, an ethynyl fragment, an azidonitrophenyl, an azidophenyl, a 3-(2-pyridyldithio)-propionamide, glyoxal and the groups of formula: 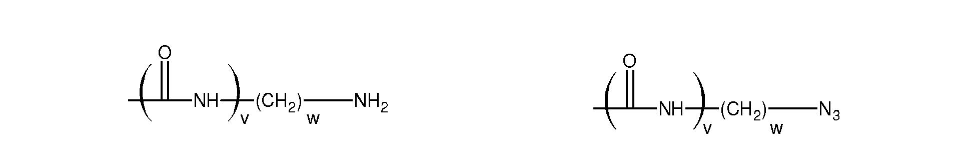 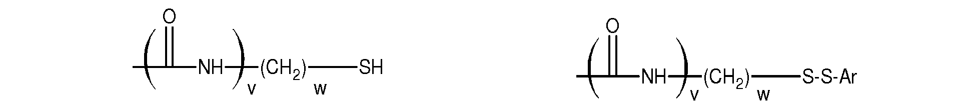 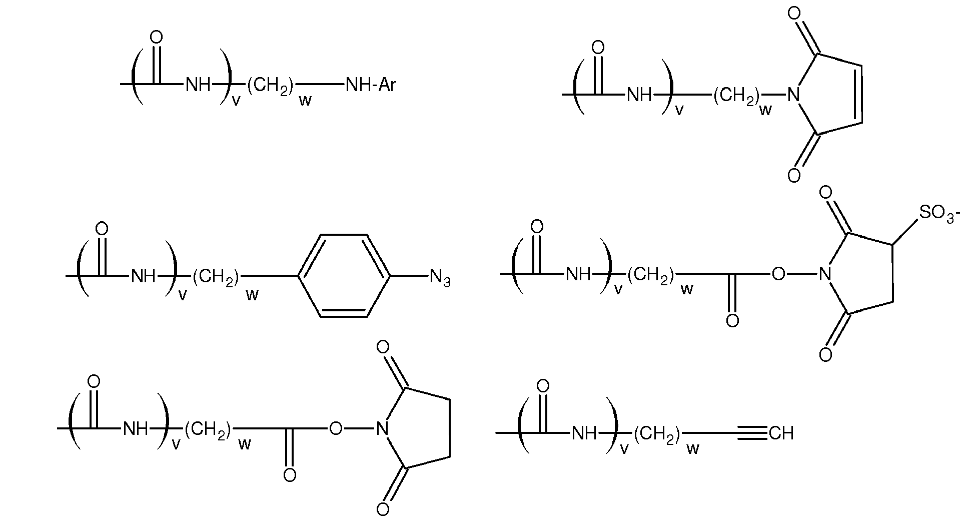 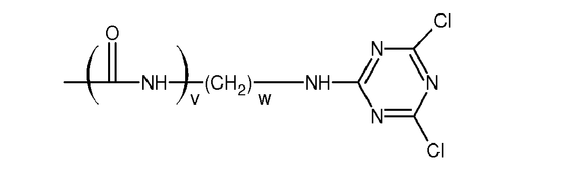 where w varies from 0 to 8 and v is equal to 0 or 1, and Ar is a 5- or 6-membered, saturated or unsaturated heterocycle comprising 1 to 3 heteroatoms, said heterocycle being optionally substituted with a halogen atom.

4. The compound according to any of the preceding claims, wherein L is a group of formula : 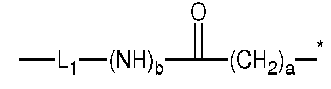 where a=b=1 and where L₁ is a C₁-C₁₀ alkylene moiety, preferably a C₂-C₆ alkylene moiety.

5. The compound according to any of the preceding claims, wherein A is -NH₂.

6. The compound according to claim 1 of formula (II):

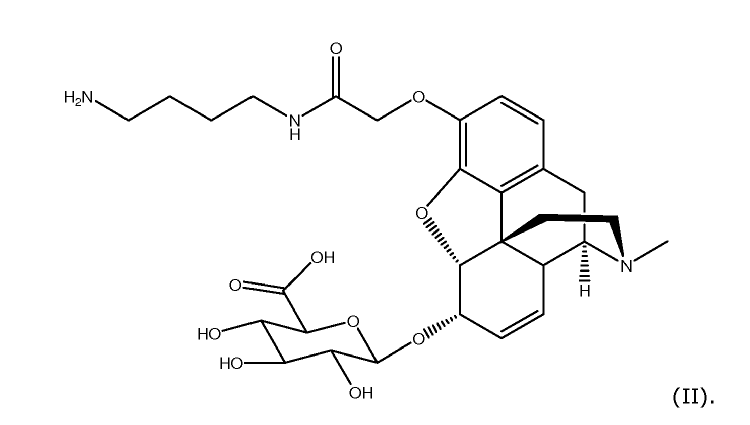

7. An immunogen comprising a compound of any one of claims 1 to 6 in which an antigenicity conferring carrier material is coupled to group A of said compound.

8. An antibody raised against the immunogen of claim 7.

9. The antibody according to claim 8, having a specificity for M6G and a cross-reactivity of less than 5% for morphine, M3G and codeine, when compared to 100% for M6G.

10. A conjugate comprising the compound of any one of claims 1 to 6 in which a detectable labeling agent is coupled to group A of said compound.

11. The conjugate according to claim 10 in which the labeling agent is selected from an enzyme, a luminescent substance and a radioactive substance.

12. A process of preparing an antibody according to Claim 8, comprising the steps of immunizing an animal, preferably a vertebrate animal, most preferably a mammalian animal, by repeated administration of an immunogen according to Claim 7; and collecting the resulting serum from the immunized animal.

13. A process of preparing an antibody according to Claim 8, comprising the steps of immunizing an animal, preferably a vertebrate animal, most preferably a mammalian animal, by repeated administration of an immunogen according to Claim 7, collecting the spleen of said animal; and fusing a mixture of lymphocytes and plasmocytes from said spleen with myelomatous cells in order to obtain hybridomas.

14. A method for detecting or determining M6G in a sample, comprising the steps of contacting the sample with a conjugate according to Claim 10 or 11 with an antibody according to Claim 8; detecting or determining bound conjugate; and deducing from a calibration curve the presence of, or the amount of M6G in the sample.

15. A kit for detecting or determining M6G, the kit including a conjugate according to Claim 10 or 11 and an antibody according to Claim 8.

16. Use of a conjugate according to Claim 10 or 11, with an antibody according to Claim 8, to detect or determine M6G in a sample.
